# EUROPÄISCHE PATENTSCHRIFT

(11) **EP 3 007 757 B1**
(45) Veröffentlichungstag und Bekanntmachung des Hinweises auf die Patenterteilung: **18.05.2022**
(21) Anmeldenummer: 14736648.8
(22) Anmeldetag: 12.06.2014
(51) Int. Cl.: A61M 39/00, A61M 39/10, F16L 19/04, F16L 47/04

(54) **VERWENDUNG EINES VERBINDUNGSELEMENTS UND VERBINDUNGSANORDNUNG**
USE OF A CONNECTING ELEMENT AND CONNECTING ASSEMBLY
UTILISATION D'UN ÉLÉMENT DE LIAISON ET ENSEMBLE DE LIAISON

(30) Priorität: 12.06.2013 DE 102013210922
(43) Veröffentlichungstag der Anmeldung: 20.04.2016
(73) Patentinhaber: W.O.M. World of Medicine GmbH, 10587 Berlin (DE)
(72) Erfinder: LIEBE, Olaf, 13055 Berlin (DE); LANGEN, Fabian, 10407 Berlin (DE); GELBERT, Nils, 12555 Berlin (DE)
(74) Vertreter: Maikowski & Ninnemann Patentanwälte Partnerschaft mbB
(86) Internationale Anmeldenummer: PCT/EP2014/062237
(87) Internationale Veröffentlichungsnummer: WO 2014/198828

(56) Entgegenhaltungen:
- DE-A1- 3 503 562
- US-A- 5 935 112
- US-A1- 2010 036 329

## Beschreibung

Die Erfindung betrifft die Verwendung eines Verbindungselements gemäß dem Oberbegriff des Anspruchs 1 und eine Verbindungsanordnung gemäß dem Oberbegriff des Anspruchs 13.

Aus dem Stand der Technik sind zur Verbindung zweier Schläuche oder anderer Hohlkörper, die von Fluiden durchströmt werden können, zahlreiche Verbindungsmöglichkeiten bekannt. Häufig werden Luer-Lock-Konnektoren eingesetzt, die teilweise mit einer Überwurfmutter zur Verschraubung versehen sind. Derartige Konnektoren dichten über die Anlagefläche zwischen der Außenseite eines konisch geformten inneren Kegels und der Außenseite eines ebenfalls konisch geformten äußeren Kegels. Dabei ist um den inneren Kegel herum ein Gewinde vorgesehen, in das der äußere Kegel durch einen entsprechend geformten Vorsprung eingeschraubt werden kann. Zusätzlich können derartige Luer-Lock-Konnektoren durch eine Überwurfmutter gesichert werden, um ein ungewolltes Öffnen zu verhindern. Der innere Kegel wird dabei auch als männlicher Luer-Lock-Anschluss und der äußere Kegel als weiblicher Luer-Lock-Anschluss bezeichnet.

Problematisch bei der Ausbildung einer derartigen Verbindung mittels eines Luer-Lock-Konnektors ist einerseits die mangelnde Dichtigkeit derartiger Verbindungen und anderseits der durch die Norm vorgegebene geringe Innendurchmesser des männlichen Luer-Lock-Anschlusses, der bei 2,5 mm liegt. Durch einen derart geringen Innendurchmesser wird die Durchflussrate durch ein gesamtes System, in dem ein solcher Luer-Lock-Konnektor eingesetzt wird, erheblich eingeschränkt.

Bei zahlreichen Instrumenten, die mit Flüssigkeiten durchströmt werden, spielt eine derartige Einschränkung des Durchflusses keine überragende Rolle, da der Innendurchmesser anderer in solchen Instrumenten verwendeter Schläuche noch geringer ist. Insbesondere bei Instrumenten, die von Gasen durchströmt werden, wären jedoch höhere Durchflussraten äußerst wünschenswert. Beispielsweise bieten Trokare einen deutlich größeren Innendurchmesser, so dass bei Systemen, in denen Trokare eingesetzt werden, regelmäßig die klassischen Luer-Lock-Verbindungen einen Engpass darstellen, der den Durchfluss des gesamten Systems bestimmt.

Aus der DE 35 03 562 A1 ist ein Quetschverbinder zum luftdichten Anschluss eines Schlauches, insbesondere eines Drainageschlauches, bekannt. Dieser Drainageschlauch ist ausweislich dieser deutschen Patentanmeldung nicht mit einem Luer-Lock-Anschluss verbunden, sondern wird ohne einen derartigen Anschluss in dem Quetschverbinder aufgenommen.

Die US 5,935,112 A beschreibt eine Ventileinrichtung, die für eine abgedichtete Durchführung eines Katheters verwendet werden kann. Dieses US-Patent beschreibt ferner, dass ein zusätzlicher Zugangsstutzen 22 an seinem Ende einen Schraubengang aufweist, damit ein üblicher Luer-Lock-Anschluss aufgenommen werden kann.

Die US 2010/0036329 A1 beschreibt eine Ventilanordnung, bei der ein Ventil durch eine in einem Gewinde geführte Überwurfmutter zusammengepresst werden kann, um selbst einen Katheter oder ein vergleichbares Gerät, das durch die Ventilanordnungen hindurchgeführt wird, abzudichten.

Der vorliegenden Erfindung liegt die Aufgabe zugrunde, eine neuartige Verwendung für ein Verbindungselement für Schläuche und andere von Fluiden durchströmbare Hohlkörper anzugeben, damit ein höhere Durchfluss als bei herkömmlichen Luer-Lock-Verbindungen erreicht werden kann und damit derartige Luer-Lock-Verbindungen ersetzt werden können. Vorzugsweise soll dabei eine vollständige Abdichtung der Verbindung erreicht und ein unbeabsichtigtes Lösen der Verbindung verhindert werden.

Diese Aufgabe wird mit der Verwendung eines Verbindungselements mit den Merkmalen des Anspruchs 1 und einer Verbindungsanordnung mit den Merkmalen des Anspruchs 13 gelöst.

Das zu der neuartigen Verwendung eingesetzte Verbindungselement dient zur Verbindung zweier von einem Fluid durchströmbarer Hohlkörper. Es weist ein erstes Verbindungsteil auf, das in fluiddichte Strömungsverbindung mit einem ersten von einem Fluid durchströmbaren Hohlkörper gebracht werden kann. Zu diesem Zweck kann das erste Verbindungsteil beispielsweise in einen derartigen Hohlkörper eingesteckt oder über einen derartigen Hohlkörper gestülpt werden. Ferner ist es möglich, Sicherungsmechanismen wie etwa das Vorsehen einer Schlauchklemme oder ein Verkleben des Verbindungsteils und des Hohlkörpers zur fluiddichten Strömungsverbindung vorzusehen.

Das Verbindungselement weist ferner ein Dichtelement auf, das zumindest teilweise in einem Innenbereich des ersten Verbindungsteils angeordnet ist. Dabei ist das Dichtelement vorzugsweise auf der dem Hohlkörper entgegengesetzten Seite des Verbindungselements in diesem angeordnet. Schließlich weist das Verbindungselement auch noch ein Sicherungselement auf, das an einer Außenseite des ersten Verbindungsteils angeordnet ist.

Das Sicherungselement kann von einer entsicherten Position in eine gesicherte Position überführt werden kann, wobei das Sicherungselement in der gesicherten Position derart auf das Dichtelement einwirkt, dass ein innerer Durchmesser des Dichtelements kleiner ist als ein erster Durchmesser des Dichtelements, wenn sich das Sicherungselement in der entsicherten Position befindet. Diese durch die Einwirkung des Sicherungselementes auf das Dichtelement bewirkte Verringerung des inneren Durchmessers des Dichtelements fällt dabei derart aus, dass eine fluiddichte Verbindung zwischen dem Innenbereich des ersten Verbindungsteils und einem Innenbereich eines zweiten Verbindungsteils bereitgestellt wird, wenn das zweite Verbindungsteil in dem Innenbereich des ersten Verbindungsteils aufgenommen ist. Mit anderen Worten ausgedrückt, umschließt das Dichtelement das zweite Verbindungsteil derart dicht, dass ein Fluid aus dem Innenbereich des ersten Verbindungsteils nur in den Innenbereich des zweiten Verbindungsteils gelangen kann, nicht jedoch auf eine Außenseite des zweiten Verbindungsteils. Falls das betrachtete Fluid nicht vom ersten Verbindungsteil zum zweiten Verbindungsteil, sondern in umgekehrter Richtung strömt, gelten die vorgenannten Ausführungen zur Fluiddichtigkeit der etablierten Verbindung selbstverständlich in analoger Weise.

Das Verbindungselement wird erfindungsgemäß zur Aufnahme eines weiblichen Luer-Lock-Anschlusses als zweitem Verbindungsteil in dem Innenbereich des ersten Verbindungsteils verwendet. Dabei wird in der gesicherten Stellung eine Dichtung über die Außenseite des weiblichen Luer-Lock-Anschlusses realisiert, wenn der weibliche Luer-Lock-Anschluss in dem Innenbereich des ersten Verbindungsteils aufgenommen ist.

Bei dieser neuartigen Verwendung ersetzt das vorliegend beschriebene Verbindungselement einen männlichen Luer-Lock-Anschluss. Dabei kommt es zu einer vollkommen anders ausgestalteten Realisierung einer Dichtung zwischen dem weiblichen Luer-Lock-Anschluss und dem vorliegend beschriebenen Verbindungselement im Vergleich zu einem herkömmlichen Luer-Lock-Konnektor. Wie oben ausgeführt, erfolgt die Dichtung bei einem herkömmlichen Luer-Lock-Konnektor nämlich durch die Anlageflächen zwischen der Außenseite des Kegels des männlichen Luer-Lock-Anschlusses und der Innenseite des Kegels des weiblichen Luer-Lock-Anschlusses. Erfindungsgemäß wird die Dichtung hingegen über die Außenseite des weiblichen Luer-Lock-Anschlusses realisiert (der am Innenbereich des weiblichen Luer-Lock-Anschlusses ausgebildete Kegel spielt für die Dichtigkeit bei dieser Erfindungsvariante überhaupt keine Rolle). Damit wird der weibliche Luer-Lock-Anschluss in ein männliches Teil umgewandelt, das in das Verbindungselement, dessen neuartige Verwendung vorliegend beansprucht wird, eingeschoben wird. Dieser für einen Fachmann absolut ungewöhnliche Rollentausch einzelner Anschlüsse resultiert letztlich in einem größeren effektiven Querschnitt, der für einen Durchfluss eines Fluids zur Verfügung steht.

Die Innenbereiche des ersten Verbindungsteils und des zweiten Verbindungsteils sind hohl ausgestaltet, so dass sie von einem Fluid durchströmt werden können. Das erste Verbindungsteil stellt also eine Verlängerung des ersten Hohlkörpers dar, die eine einfache Verbindung des ersten Hohlkörpers mit einem zweiten Hohlkörper ermöglicht, sofern der zweite Hohlkörper mit einem entsprechenden zweiten Verbindungsteil ausgestattet ist. Das Verbindungselement und das zweite Verbindungsteil stellen folglich miteinander verbindbare Gegenstücke dar.

Anstatt der bisher üblichen starren Schraubverbindungen wird durch das neuartige Verbindungselement eine flexible Steckverbindung realisiert. Das Verbindungselement dient gleichsam als dehnbarer Stecker und erlaubt das universelle Verbinden unterschiedlicher Anschlüsse, wobei gleichzeitig ein deutlich größerer Innendurchmesser realisiert wird, als dies bei aus dem Stand der Technik bekannten Lösungen der Fall ist. Die Dichtung zwischen den einzelnen Komponenten erfolgt dabei über die Außenfläche eines weiblichen Verbindungsstücks (nämlich dem zweiten Verbindungsteil), das in das beschriebene Verbindungselement eingesteckt wird. Gesichert wird die Verbindung durch das Sicherungselement, das beispielsweise als auf der Innenseite konisch geformte Kappe ausgestaltet sein kann und das beispielsweise durch eine Verdrehung das Dichtelement komprimiert.

Die Verringerung des inneren Durchmessers des Dichtelements kann durch einen direkten Kontakt zwischen dem Sicherungselement und dem Dichtelement oder durch einen indirekten Kontakt zwischen dem Sicherungselement und dem Dichtelement realisiert werden. So ist es beispielsweise denkbar, dass das Sicherungselement auf das erste Verbindungsteil einwirkt, wodurch es zu einer Verringerung des inneren Durchmessers des ersten Verbindungsteils kommt. Eine derartige Verringerung des inneren Durchmessers des ersten Verbindungsteils würde dann in einer Verringerung des inneren Durchmessers des Dichtelementes resultieren. Ferner ist es aber auch möglich, dass das Sicherungselement direkt das Dichtelement zusammendrückt, so dass der innere Durchmesser des Dichtelements verringert wird.

Dabei ist es nicht erforderlich, dass der innere Durchmesser des Dichtelements oder der innere Durchmesser des Verbindungsteils über die gesamte Länge des Dichtelements bzw. des Verbindungsteils gleich groß ist. Vielmehr ist es denkbar, dass der innere Durchmesser des Verbindungsteils oder des Dichtelements über die Länge des jeweiligen Elements variiert. Der Begriff "ein innerer Durchmesser" bezeichnet dabei den inneren Durchmesser an einer ausgewählten Stelle des Verbindungsteils oder des Dichtelements. Eine Verringerung des inneren Durchmessers an einer ausgewählten Stelle kann die inneren Durchmesser an anderen ausgewählten Stellen des Verbindungsteils oder des Dichtelements unbeeinflusst lassen.

Vorzugsweise ist das Dichtelement derart ausgestaltet und dimensioniert, dass es an der Innenseite des Verbindungsteils derart dicht anliegt, dass kein Fluid zwischen das Dichtelement und die Innenseite des Verbindungsteils eintreten kann. Das Dichtelement kann insbesondere in das erste Verbindungsteil eingeklebt sein. Ferner ist zumindest in einem Abschnitt des Verbindungsteils der gesamte Innenumfang des Verbindungsteils von dem Dichtelement überdeckt. Mit anderen Worten ausgedrückt, entspricht die Innenkontur eines Abschnitts des Verbindungsteils vorzugsweise der Außenkontur eines Abschnitts des Dichtelements. Wenn der Innenbereich des Verbindungsteils einen Querschnitt eines Kreiszylinders aufweist, ist das Dichtelement vorzugsweise zumindest abschnittsweise als Hohlkreiszylinder ausgebildet.

Vorzugsweise ist das Sicherungselement derart ausgestaltet, dass es das erste Verbindungsteil zumindest abschnittsweise überdeckt. Dies ermöglicht eine leichte Zugänglichkeit des Sicherungselements von außen, die wiederum eine leichte Betätigung des Sicherungselementes gestattet.

In einer weiteren Variante überdeckt das Sicherungselement einen gesamten Außenumfang des ersten Verbindungsteils zumindest in einem Abschnitt des ersten Verbindungsteils. Beispielsweise kann das Sicherungselement als Kappe ausgeführt sein, die an einem Abschnitt des Verbindungsteils, der insbesondere ein endständiger Abschnitt sein kann, über das Verbindungsteil gesteckt ist. Eine derartige, den gesamten Außenumfang des Verbindungsteils überdeckende Ausgestaltung des Sicherungselements gestattet eine besonders einfache Handhabung des Sicherungselements, da dieses dann von einem Benutzer leicht gedreht oder lateral verschoben werden kann.

In einer weiteren Ausgestaltung des Verbindungselements stehen das erste Verbindungsteil und das Sicherungselement über ein Gewinde miteinander in Eingriff. Mittels eines derartigen Gewindes ist es möglich, dass eine relativ zum Verbindungsteil erfolgende Bewegung des Sicherungselements entlang des Gewindes realisiert werden kann. Auf diese Weise ist es dann möglich, das Sicherungselement von seiner entsicherten Position in seine gesicherte Position zu überführen (und umgekehrt). Das Gewinde kann beispielsweise in einem Abschnitt des Verbindungsteils ausgeführt sein. Vorzugsweise handelt es sich bei diesem Abschnitt um denjenigen Abschnitt, den das Sicherungselement überdeckt. Ferner kann im Sicherungselement ein Stift, eine Nase oder ein anderer Vorsprung vorgesehen sein, der in dem Gewinde geführt wird. Grundsätzlich ist es auch denkbar, das Gewinde im Sicherungselement vorzusehen und einen Führungsstift, einen Vorsprung oder eine Nase am Verbindungsteil zur Führung in dem Gewinde vorzusehen.

In einer Variante ist es denkbar, dass das Sicherungselement nur von seiner entsicherten Stellung in seine gesicherte Stellung überführt werden kann. Auf diese Weise würde man die zwischen den miteinander zu verbindenden Hohlkörpern herzustellende Verbindung genau einmal herstellen können. Sie würde dann dauerhaft erhalten bleiben. Dies kann bei einigen Anwendungen von Vorteil sei. Häufiger wird jedoch eine reversible bzw. lösbare Verbindung zweier Hohlkörper gewünscht. Daher kann das Sicherungselement in einer weiteren alternativen Ausgestaltung des Verbindungselements auch von der gesicherten Stellung wieder zurück in die entsicherte Stellung überführt werden. Auf diese Weise kann also eine bereits hergestellte Verbindung zwischen zwei Hohlkörpern wieder gelöst werden.

Vorzugsweise kann es vorgesehen sein, dass das Sicherungselement in seiner gesicherten Stellung verrastet, so dass eine Überführung des Sicherungselementes von der gesicherten Stellung in seine entsicherte Stellung nur durch einen erhöhten Kraftaufwand möglich ist. Eine derartige Ausgestaltung beugt effektiv einem unerwünschten Öffnen der hergestellten Verbindung vor.

In einer weiteren Variante übt das Sicherungselement in seiner gesicherten Position eine Klemmkraft auf das Dichtelement und, wenn in dem Innenbereich des ersten Verbindungsteils ein zweites Verbindungsteil aufgenommen ist, auch auf dieses zweite Verbindungsteil aus. Das heißt, die dichte Verbindung zwischen dem Verbindungselement auf der einen Seite und dem zweiten Verbindungsteil auf der anderen Seite wird durch eine entsprechende Klemmkraft des Sicherungselementes realisiert.

In einer weiteren Variante ist das Sicherungselement zumindest abschnittsweise konisch ausgestaltet. Dabei ist vorzugsweise die Innenseite des Sicherungselementes in einem Abschnitt konisch ausgeformt. Auf diese Weise ist es möglich, durch eine Relativverschiebung des Sicherungselementes gegenüber dem Verbindungsteil einen unterschiedlich großen maximal möglichen Außendurchmesser für Elemente bereitzustellen, die mit dem Sicherungselement in Kontakt kommen. Hierbei kann es sich insbesondere um das Dichtelement oder das Verbindungsteil handeln. Durch eine entsprechende konische Ausgestaltung eines Innenbereichs des Sicherungselements ist es also möglich, eine Klemmkraft bereitzustellen, die eine Verringerung des Innendurchmessers des Dichtelements bewirkt.

In einer weiteren Ausgestaltung ist das Sicherungselement bei bestimmungsgemäßer Benutzung des Verbindungselementes stets mit dem Verbindungsteil verbunden. Diese Verbindung kann beispielsweise über ein Gewinde und einen in dem Gewinde geführten Vorsprung erfolgen. Zur Realisierung dieser Variante können das Sicherungselement und das Verbindungsteil als separate Bauteile hergestellt werden und vor einer Freigabe des Verbindungselements zur Benutzung miteinander verbunden werden. Zu diesem Zweck kann das Sicherungselement beispielsweise mit einer entsprechenden Rastnase über einen Vorsprung des Verbindungsteils gedrückt werden, so dass es anschließend in einem Gewinde des Verbindungsteils geführt werden kann, nicht jedoch ohne übermäßigen Kraftaufwand vom Verbindungsteil getrennt werden kann. Dies erleichtert den Vertrieb und die Benutzung eines entsprechend ausgestalteten Verbindungselements.

In einer weiteren Variante ist ein Abschnitt des Dichtelements außerhalb des Innenbereichs des ersten Verbindungsteils angeordnet. Vorzugsweise handelt es sich bei diesem Abschnitt um einen umlaufenden, wulstartigen Vorsprung, der sich entlang des gesamten Umfangs des Dichtelements erstreckt. Mittels eines solchen Vorsprungs ist es auf besonders einfache Art und Weise möglich, eine definierte Position vorzugeben, in der sich das Dichtelement in dem Verbindungsteil zum bestimmungsgemäßen Gebrauch des Verbindungselements befinden soll. Denn durch einen entsprechenden Vorsprung kann die maximal mögliche Einstecktiefe des Dichtelements in dem Verbindungsteil definiert werden. Dies ist insbesondere dann der Fall, wenn sich der Vorsprung zur Außenseite des Dichtelementes erstreckt. Wenn sich der Vorsprung zur Innenseite des Dichtelementes erstreckt, bietet er den Vorteil, dass ein in das Dichtelement (und damit in den Innenbereich des Verbindungsteils) eingestecktes zweites Verbindungsteil hinter dem entsprechenden Vorsprung verrasten kann.

Die maximal mögliche Einstecktiefe des Dichtelementes in dem ersten Verbindungsteil kann insbesondere auch durch eine entsprechende Ausgestaltung des ersten Verbindungsteils definiert werden. Vorzugsweise ist das erste Verbindungsteil dabei derart ausgestaltet, dass es einen Absatz bildet, der zur Anlage des Dichtelementes im Innenbereich des ersten Verbindungsteils dient. Der Absatz weist dabei eine solche Tiefe auf, dass sich der innere Durchmesser des ersten Verbindungsteils zwischen einem Bereich, in dem das Dichtelement angeordnet ist, und einem Bereich, in dem das Dichtelement nicht angeordnet ist, im Wesentlichen nicht unterscheidet. Eine derartige Ausgestaltung erlaubt ein besonders vorteilhaftes Strömen eines Fluids durch das erste Verbindungsteil hindurch, da im Wesentlichen keine Verwirbelungen entstehen.

In einer weiteren Variante wird der außerhalb des Innenbereichs des ersten Verbindungsteils angeordnete Abschnitt des Dichtelements direkt vom Sicherungselement kontaktiert, wenn sich dieses in seiner gesicherten Position befindet. Das heißt, das Sicherungselement drückt in seiner gesicherten Position direkt gegen denjenigen Abschnitt des Dichtelements, der nicht vollständig im Innenbereich des ersten Verbindungsteils aufgenommen ist. Dadurch kommt es zu einer Verringerung des Durchmessers des Dichtelements, die in einer dichten Verbindung mit dem zweiten Verbindungsteil resultiert, wenn ein solches zweites Verbindungsteil in den Innenbereich des ersten Verbindungsteils eingesteckt ist.

In einer weiteren Variante ist der außerhalb des Innenbereichs des ersten Verbindungsteils angeordnete Abschnitt des Dichtelements dafür vorgesehen und eingerichtet, einen Vorsprung eines zweiten Verbindungsteils hinterschnittartig zu umgreifen, wenn ein solches zweites Verbindungsteil in dem Innenbereich des ersten Verbindungsteils aufgenommen ist. Dies ist insbesondere dann möglich, wenn das Dichtelement auf seiner Innenseite einen umlaufenden wulstartigen Vorsprung aufweist, der hinter einen entsprechenden Vorsprung des zweiten Verbindungsteils greifen kann. Bei der derartigen Ausgestaltung sorgt eine von dem Sicherungselement in seiner gesicherten Position ausgeübte Klemmkraft auf das Dichtelement nicht nur für eine dichte Verbindung zwischen dem ersten Verbindungsteil und dem zweiten Verbindungsteil, sondern gleichzeitig auch für einen sicheren Sitz des zweiten Verbindungsteils im Verbindungselement, der nicht ohne Überführung des Sicherungselements in seine entsicherte Position gelöst werden kann.

Wie bereits erwähnt, muss der innere Durchmesser des ersten Verbindungsteils nicht notwendigerweise über die gesamte Länge des ersten Verbindungsteils konstant sein. Vorzugsweise beträgt der kleinste innere Durchmesser des ersten Verbindungsteils rund 3 mm bis rund 15 mm, insbesondere rund 4 mm bis rund 14 mm, insbesondere rund 5 mm bis rund 13 mm, insbesondere rund 6 mm bis rund 12 mm, insbesondere rund 7 mm bis rund 11 mm und ganz besonders rund 8 mm bis rund 10 mm. Auf diese Weise ist sichergestellt, dass das Verbindungselement einen höheren Durchfluss als herkömmliche Luer-Lock-Anschlüsse ermöglicht, denn der innere Durchmesser männlicher Luer-Lock-Anschlüsse beträgt lediglich 2,5 mm. Wegen der existierenden Norm zu derartigen Luer-Lock-Anschlüssen gab es für einen Fachmann bisher keinerlei Veranlassung, den Innendurchmesser derartiger Verbindungen zu vergrößern.

Vorzugsweise wird der kleinste innere Durchmesser des ersten Verbindungsteils in demjenigen Abschnitt des ersten Verbindungsteils bestimmt, der von einem Fluid durchströmt wird, wenn eine entsprechende Verbindung zwischen dem Verbindungselement und einem zweiten Verbindungsteil hergestellt ist. Ferner wird als kleinster innerer Durchmesser des ersten Verbindungsteils auch ein durch ein Dichtelement verringerter innerer Durchmesser des ersten Verbindungsteils angesehen, da dieser für die Strömungs- bzw. Durchflusseigenschaften des ersten Verbindungsteils ebenfalls von entscheidender Bedeutung ist. Nicht berücksichtigt beim inneren Durchmesser des ersten Verbindungsteils wird hingegen ein innerer Durchmesser des Dichtelements, der sich in einem Abschnitt des Dichtelements ergibt, der außerhalb des ersten Verbindungsteils und/oder außerhalb eines Bereichs, der bei einer hergestellten Verbindung zwischen dem Verbindungselement und einem zweiten Verbindungsteil von einem Fluid durchströmt wird, liegt.

Die der Erfindung zugrunde liegende Aufgabe wird auch durch eine Verbindungsanordnung mit den Merkmalen des Anspruchs 13 gelöst. Eine derartige Verbindungsanordnung weist ein Verbindungselement gemäß den vorherigen Erläuterungen auf. Ferner ist ein zweites Verbindungsteil vorgesehen, das in einem Innenbereich eines ersten Verbindungsteils des Verbindungselementes aufgenommen ist und das in fluiddichte Strömungsverbindung mit einem zweiten von einem Fluid durchströmbaren Hohlkörper gebracht werden kann. Bei dem zweiten Verbindungsteil handelt es sich um einen weiblichen Luer-Lock-Anschluss. Damit wird in Abwandlung herkömmlicher Luer-Lock-Konnektoren eine neuartige Verbindungsanordnung bereitgestellt, die einen signifikant größeren Durchfluss als herkömmliche Luer-Lock-Konnektoren gestattet.

Eine derartige Verbindungsanordnung ermöglicht eine fluiddichte Verbindung zwischen einem ersten Hohlkörper, der mit dem ersten Verbindungsteil des Verbindungselements verbunden ist, und dem zweiten Hohlkörper, der mit dem zweiten Verbindungsteil verbunden ist. Je nach spezifischer Ausgestaltung des Sicherungselements und des Verbindungsteils des Verbindungselements kann dabei eine unlösbare oder lösbare Verbindung zwischen dem ersten Hohlkörper und dem zweiten Hohlkörper hergestellt werden.

Bevorzugte Ausgestaltungen des beschriebenen Verbindungselementes bzw. der Verwendung des Verbindungselements sind in analoger Weise auf die beschriebene Verbindungsanordnung übertragbar, und umgekehrt.

Weitere Details und Einzelheiten der Erfindung werden nachfolgend im Zusammenhang mit den angehängten Figuren erläutert. Es zeigen:
- Figur 1: eine Explosionsdarstellung eines Ausführungsbeispiels eines Verbindungselements,
- Figur 2: eine seitliche Ansicht auf das Verbindungselement der Figur 1,
- Figur 3A: eine teilweise geschnittene Ansicht des Verbindungselements der Figur 1, wobei sich eine Kappe in ihrer entsicherten Stellung befindet,
- Figur 3B: eine teilweise geschnittene Ansicht des Verbindungselements der Figur 1, wobei sich eine Kappe in ihrer gesicherten Stellung befindet,
- Figur 4: eine teilweise geschnittene Ansicht eines Ausführungsbeispiels einer Verbindu ngsanord nu ng,
- Figur 5: eine seitliche Ansicht auf die Verbindungsanordnung der Figur 4,
- Figur 6: eine graphische Darstellung des gemessenen Durchflusses in Abhängigkeit der erzeugten Flussrate in einem System, das mit einem Ausführungsbeispiel eines Verbindungselements ausgestattet ist,
- Figur 7: eine weitere graphische Darstellung des gemessenen Durchflusses in Abhängigkeit der erzeugten Flussrate in einem System, das mit einem Ausführungsbeispiel eines Verbindungselementes ausgestattet ist,
- Figur 8: eine graphische Darstellung des gemessenen Staudrucks in Abhängigkeit der erzeugten Flussrate in einem System, das mit einem Ausführungsbeispiel eines Verbindungselementes ausgestattet ist, und
- Figur 9: eine graphische Darstellung des Quotienten aus Staudruck und Durchfluss in Abhängigkeit der erzeugten Flussrate in einem System, das mit einem Ausführungsbeispiel eines Verbindungselementes ausgestattet ist.

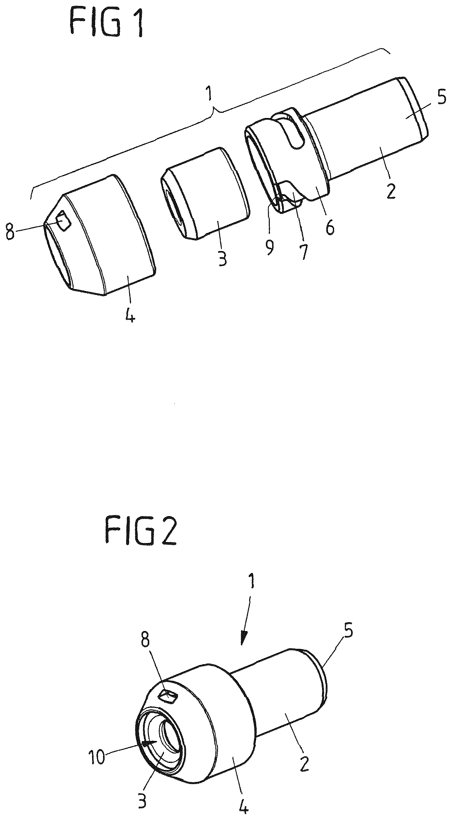

Die Figur 1 zeigt eine Explosionsdarstellung eines Adapters 1 als Verbindungselement, der aus einem ersten Adapterteil 2 als erstem Verbindungsteil, einer Dichtung 3 als Dichtelement und einer Kappe 4 als Sicherungselement besteht. Das Adapterteil 2 ist dafür vorgesehen und eingerichtet, mit einem Schlauch oder einem anderen Hohlkörper, der von einer Flüssigkeit oder einem Gas durchströmt werden kann, verbunden zu werden. Zu diesem Zwecke wird das Adapterteil 2 in einen entsprechenden Schlauch gesteckt oder über einen entsprechenden Schlauch gestülpt bzw. gezogen. Die entsprechende Verbindung des Adapterteils 2 mit einem derartigen Schlauch erfolgt an einem ersten Ende 5 des Adapterteils 2.

Im bestimmungsgemäßen Betrieb des Adapters 1 ist die Dichtung 3 größtenteils in einem Innenbereich des Adapterteils 2 aufgenommen.

Das Adapterteil 2 weist an einem zweiten Ende 6, das dem ersten Ende 5 gegenüberliegt, ein Gewinde 7 auf. Dieses Gewinde ist dafür vorgesehen und eingerichtet, dass die Kappe 4 sich entlang des Gewindes 7 bewegen kann. Zu diesem Zweck ist die Kappe 4 mit einer Rastnase 8 versehen, die in das Gewinde 7 eingreift und dank des Gewindes 7 geführt werden kann, wenn die Kappe 4 gegenüber dem Adapterteil 2 gedreht wird. Das Adapterteil 2 weist zudem an einem Ende 9 des Gewindes 7 einen Vorsprung auf, über den die Rastnase 8 gedrückt werden muss, um die Kappe 4 auf das Adapterteil 2 zu setzen. Dieser Vorsprung dient gleichzeitig dazu, dass die Kappe 4 nicht unbeabsichtigt von dem Adapterteil 2 entfernt werden kann. So ist es auf diese Weise möglich, das Adapterteil 2 mit eingesetzter Dichtung 3 und aufgesteckter Kappe 4 als eine Einheit zu vertreiben.

Dies ist in der Figur 2 dargestellt, die den zusammengebauten Zustand des Adapters 1 wiedergibt. In dieser Darstellung ist zu sehen, wie die Kappe 4 einen Abschnitt des Adapterteils 2 überdeckt, und zwar entlang dessen gesamten Außenumfangs. Ferner ist in dieser Darstellung zu sehen, wie die Dichtung 3 in einem Innenbereich des Adapterteils 2 aufgenommen ist, da lediglich ein oberer Randbereich der Dichtung 3 durch eine Öffnung 10, die in der Kappe 4 ausgebildet ist, zu sehen ist. Die Öffnung 10 in der Kappe 4 ist dabei derart dimensioniert, dass ein weiteres Verbindungsteil unproblematisch durch die Öffnung 10 in den Innenbereich des Adapterteils 2 eingeschoben werden kann.

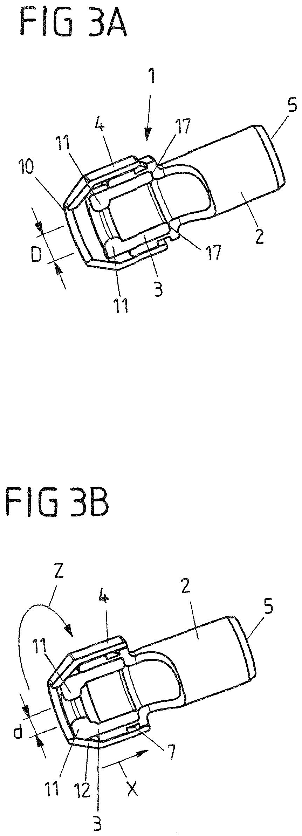

Die Figur 3A zeigt eine teilweise geschnittene Ansicht durch das Adapterteil 1, das bereits in den Figuren 1 und 2 dargstellt war. In dieser Darstellung befindet sich die Kappe 4 in einer entsicherten Zustellung, in der die Kappe 4 locker auf dem Adapterteil 2 sitzt, ohne eine Kraft auf die Dichtung 3 auszuüben. Dadurch resultiert ein erster Durchmesser D der Dichtung 3, der zwischen den Innenseiten einer umlaufenden Wulst 11 gebildet ist. Diese umlaufende Wulst 11 befindet sich dabei an einem Ende der Dichtung 3, die der Öffnung 10 der Kappe 4 zugewandt ist. Die umlaufende Wulst 11 verringert den inneren Durchmesser der Dichtung 3 gegenüber dem inneren Durchmesser der Dichtung 3 in Bereichen, in denen keine umlaufende Wulst 11 ausgebildet ist.

Die Dichtung 3 liegt in einem Innenbereich des Adapterteils 2 an einem Absatz 17 auf, der die maximal mögliche Einschubtiefe der Dichtung 3 in das Adapterteil 2 vorgibt. Gleichzeitig ist der Absatz dabei so tief ausgestaltet, dass ein innerer Durchmesser im Innenbereich des Adapterteils 2 im Wesentlichen gleich groß bleibt, unabhängig davon, ob man einen Abschnitt des Adapterteils 2 betrachtet, in dem die Dichtung 3 angeordnet ist, oder einen Abschnitt des Adapterteils 2, in dem keine Dichtung angeordnet ist.

Die Figur 3B zeigt nun das Adapterteil 1, wobei die Kappe 4 von der entsicherten Stellung in die gesicherte Stellung überführt wurde. Diese Überführung erfolgt durch ein Drehen entlang einer Richtung, die durch den mit Z bezeichneten Pfeil gekennzeichnet ist. Durch diese Drehbewegung kommt es zu einer Lateralverschiebung der Kappe 4 in Richtung des mit X bezeichneten Pfeils. Das heißt, durch eine Drehung der Kappe 4 im Gewinde 7 des Adapterteils 2 kommt es zu einer Lateralverschiebung der Kappe 4 entlang des Adapterteils 2. Diese Lateralverschiebung bewirkt, dass ein konisch ausgeformter Bereich 12 der Kappe 4 in Kontakt mit der Wulst 11 der Dichtung 3 tritt und durch die entsprechende konische Ausbildung die Wulst 11 zusammendrückt. Dadurch verringert sich der im Bereich der Wulst 11 der Dichtung 3 ausgebildete Durchmesser D in der entsicherten Stellung der Kappe 4 in den inneren Durchmesser d, der geringer ist als der Durchmesser D.

Die Kappe 4 wirkt also mit einer Klemmkraft auf die Wulst 11 der Dichtung 3 ein und kann so Gegenstände, die von der Wulst 11 der Dichtung 3 aufgenommen sind, ebenfalls verklemmen. Auf diese Weise lassen sich Gegenstände im Innenbereich des Adapterteils 2 sichern und über die Dichtung 3 zugleich abdichten.

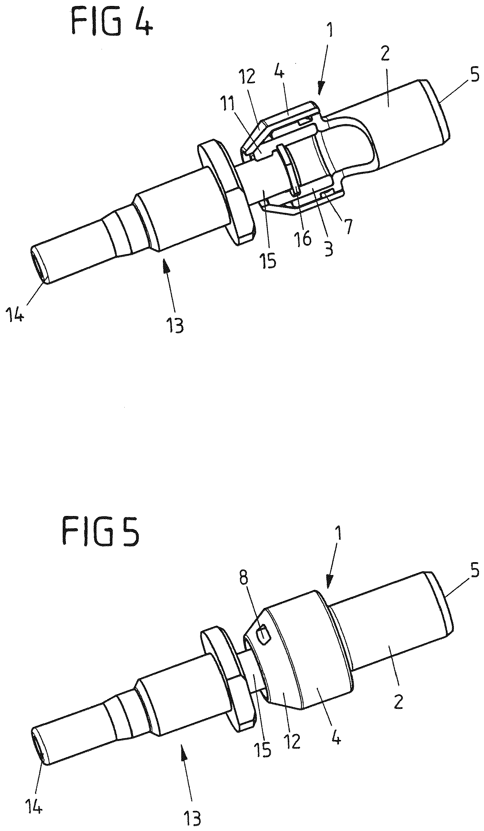

Die Figur 4 zeigt eine teilweise geschnittene Ansicht einer Verbindungsanordnung, bei der ein zweites Adapterteil 13 als zweites Verbindungsteil in das erste Adapterteil 2 aufgenommen ist. Das zweite Adapterteil 13 ist an seinem dem ersten Adapterteil 2 gegenüberliegenden Ende 14 dafür vorgesehen und eingerichtet, mit einem Schlauch oder einem anderen Hohlkörper, der von einer Flüssigkeit oder einem Gas durchströmt werden kann, verbunden zu werden. Wenn ein entsprechender Schlauch an das erste Ende 14 des zweiten Adapterteils 13 angeschlossen ist und auch ein entsprechender Schlauch an das erste Ende 5 des ersten Adapterteils 2 angeschlossen ist, werden diese beiden Schläuche durch die aus dem Adapter 1 und dem zweiten Adapterteil 13 bestehende Verbindungsanordnung miteinander verbunden.

Dabei sorgt der Adapter 1 dafür, dass das zweite Verbindungsteil 13 sicher im Innenbereich des ersten Adapterteils 2 aufgenommen ist. Hierzu wird ein zweites Ende 15 des zweiten Adapterteils 13 in den Innenbereich des ersten Adapterteils 2 eingeführt. Dieses zweite Ende 15 des zweiten Adapterteils 13 weist im Ausführungsbeispiel der Figur 4 einen umlaufenden Vorsprung 16 auf, der von der Wulst 11 der Dichtung 3 hinterschnittartig umgriffen wird, wenn das zweite Adapterteil 13 in seiner bestimmungsgemäßen Position im Innenbereich des ersten Adapterteils 2 angeordnet ist. Nun kann die Kappe 4 entlang des Gewindes 7 gedreht werden, um auf diese Weise eine Lateralverschiebung der Kappe 4 gegenüber dem ersten Adapterteil 2 zu erreichen. Dadurch klemmt die Kappe 4 mit ihrem konischen Bereich 12 die Wulst 11 der Dichtung 3 gegen das zweite Ende 15 des zweiten Adapterteils 13 und sichert das zweite Adapterteil 13 somit in fluiddichter Weise im Innenbereich des ersten Adapterteils 2.

Wenn die Verbindung zwischen dem Adapter 1 und dem zweiten Adapterteil 13 wieder gelöst werden soll, wird die Kappe 4 in entgegengesetzter Richtung auf dem Gewinde 7 bewegt, so dass die Klemmkraft zwischen dem konischen Abschnitt 12 der Kappe 4 und der Wulst 11 der Dichtung 3 zuerst reduziert und schließlich ganz aufgehoben wird. Anschließend kann das zweite Adapterteil 13 wieder aus dem Innenbereich des ersten Adapterteils 2 entfernt werden.

Beim zweiten Adapterteil 13 kann es sich insbesondere um einen weiblichen Luer-Lock-Anschluss handeln. Gemäß dem Stand der Technik würde eine Schlauchverbindung unter Einsatz eines derartigen weiblichen Luer-Lock-Anschlusses dadurch erfolgen, dass ein männlicher Luer-Lock-Anschluss in den weiblichen Luer-Lock-Anschluss geschoben würde. Die Verbindung würde durch ein Schraubgewinde gesichert werden. Die Dichtung würde dabei im Innenbereich des weiblichen Luer-Lock-Anschlusses über die Anlagefläche zum männlichen Luer-Lock-Anschluss erfolgen. Die vorliegend beschriebene Lösung sieht eine vollkommen andere Dichtungsweise vor, denn hier erfolgt eine Dichtung auf der Außenseite eines entsprechenden weiblichen Luer-Lock-Anschlusses durch die Wulst 11 der Dichtung 3. Dadurch steht einem durch das zweite Verbindungsteil 13 und das erste Verbindungsteil 2 strömenden Fluid ein weitaus größerer Querschnitt zur Verfügung, als dies bei klassischen Luer-Lock-Anschlüssen der Fall ist.

Die Figur 5 zeigt die Verbindungsanordnung der Figur 4 in einer ungeschnittenen seitlichen Darstellung. Um unnötige Längen zu vermeiden, wird zur Beschreibung der Figur 5 auf die obigen Erläuterungen zu den anderen Figuren Bezug genommen.

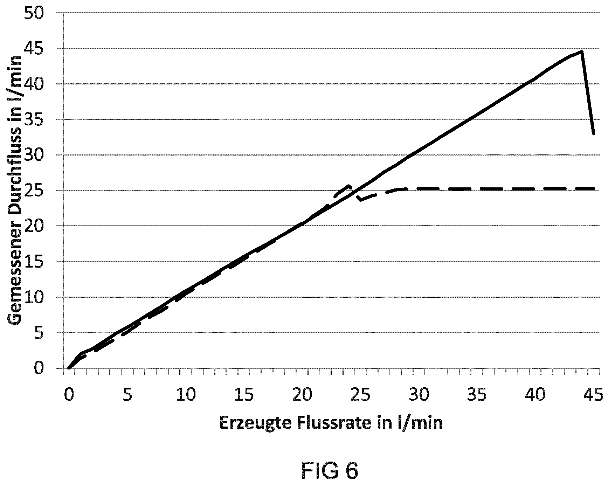

Die vorteilhaften Durchflusseigenschaften des beschriebenen Verbindungselementes werden nachfolgen anhand einer beispielhaften Versuchsanordnung im Zusammenhang mit den Figuren 6 bis 9 näher erläutert. Mit einem Insufflator des Typs F114 wurde ein Gasfluss unterschiedlicher Stärke erzeugt und durch einen 3 m langen PVC-Schlauch geleitet. Am Ende dieses Schlauchs war ein Ausführungsbeispiel eines Verbindungselements montiert, das mit einem Messgerät des Typs HRH 80 verbunden war. Dieses Messgerät kann den Durchfluss, der sich am Ausgang des Verbindungselements einstellt, bestimmen. Zudem ist dieses Messgerät in der Lage, einen Druck zu bestimmen. Zu diesem Zweck wurde eine entsprechende Verbindung zwischen der Drucksonde des Messgerätes und einem vor dem Verbindungselement angeordneten T-Stück hergestellt, um so den sich vor dem Verbindungselement ergebenden Staudruck zu ermitteln.

Mittels dieses Versuchsaufbaus erzielte Messergebnisse sind in den Figuren 6 bis 9 dargestellt. Die Figur 6 zeigt einen Vergleich des gemessenen Durchflusses aus dem Versuchssystem, wobei sich die durchgezogene Kurve auf ein Ausführungsbeispiel eines Verbindungselementes gemäß der vorliegenden Erfindung bezieht, während die gestrichelte Kurve einen aus dem Stand der Technik bekannten männlichen Standard-Luer-Lock-Anschluss betrifft (Referenzmessung).

Wie aus der Figur 6 zu ersehen ist, steigt der gemessene Durchfluss beim neuartigen Verbindungselement bis zu einer erzeugten Flussrate von rund 43 Litern pro Minute kontinuierlich und proportional zur erzeugten Flussrate an. Erst dann ist die Leistungsfähigkeit des Systems erreicht, wodurch es zu einem signifikanten Abbruch des gemessenen Durchflusses kommt. Bei dem aus dem Stand der Technik bekannten System ist hingegen bereits bei rund 23 Litern pro Minute die maximal mögliche Systemleistung erreicht. Auch wenn dann die erzeugte Flussrate des durch das System geleiteten Gases weiter erhöht wird, kommt es zu keiner Erhöhung der gemessenen Flussrate. Aufgrund des geringen Durchmessers eines männlichen Luer-Lock-Anschlusses kann ein Durchfluss von rund 25 Litern pro Minute nicht überschritten werden.

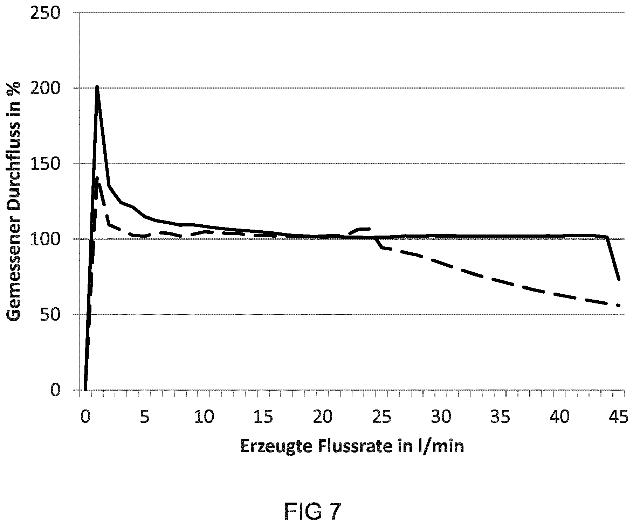

Die Figur 7 zeigt die Messwerte der Figur 6 in einer relativen Darstellung. So wird der gemessene Durchfluss in Prozent der erzeugten Flussrate aufgetragen. Wiederum betrifft die durchgezogene Kurve das neuartiges Verbindungselement gemäß dem Ausführungsbeispiel, während die gestrichelte Kurve als Referenzmessung einen aus dem Stand der Technik bekannten männlichen Luer-Lock-Anschluss betrifft. Aus dieser Darstellung ist gut zu sehen, dass beim neuartigen Verbindungselement bis zu einer erzeugten Flussrate von rund 43 Litern pro Minute ein Durchfluss gemessen werden kann, der im Wesentlichen der erzeugten Flussrate entspricht. Die Abweichungen nach oben bei geringen erzeugten Flussraten beruhen auf Messungenauigkeiten des Systems. Ferner ist aus dieser Darstellung gut ersichtlich, dass beim herkömmlichen Luer-Lock-Anschluss bereits bei etwa 23 Litern pro Minute erzeugter Flussrate die 100%-Marke unterschritten wird und bei höheren Flussraten nur noch ein deutlich unter 100 % liegender Durchfluss gemessen werden kann, der sich bei einer Flussrate von etwa 45 Litern pro Minute auf fast 50 % halbiert.

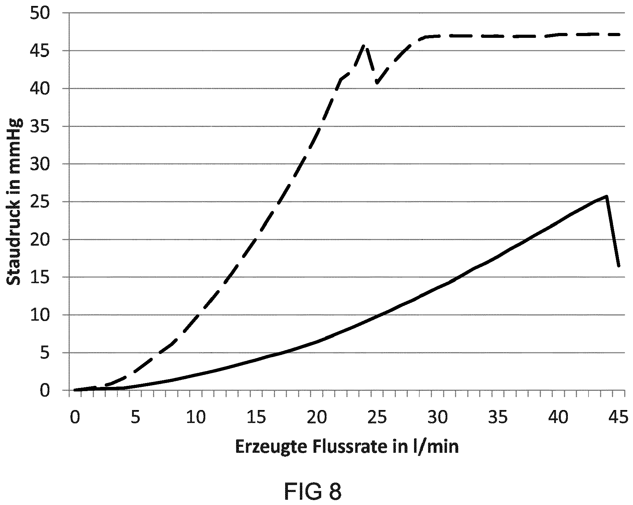

In der Figur 8 wird der vor dem jeweils eingesetzten Adapter gemessene Staudruck gegenüber der erzeugten Flussrate aufgetragen. Die durchgezogene Kurve zeigt wieder Messwerte für das Ausführungsbeispiel des Verbindungselements gemäß der vorliegenden Erfindung, während die gestrichelte Kurve Messwerte wiedergibt, die mit einem aus dem Stand der Technik bekannten männlichen Luer-Lock-Anschluss ermittelt wurden. Wie aus der Graphik der Figur 8 gut zu ersehen ist, steigt der Staudruck bei dem aus dem Stand der Technik bekannten System bereits bei geringen erzeugten Flussraten signifikant an. Demgegenüber kommt es bei dem neuartigen Verbindungselement lediglich zu einem leichten Anstieg des Staudrucks bis zu einer Flussrate von etwa 43 Litern pro Minute. Dann kommt es zur Auslastung des Systems, die in einem Abfall des gemessenen Staudrucks resultiert.

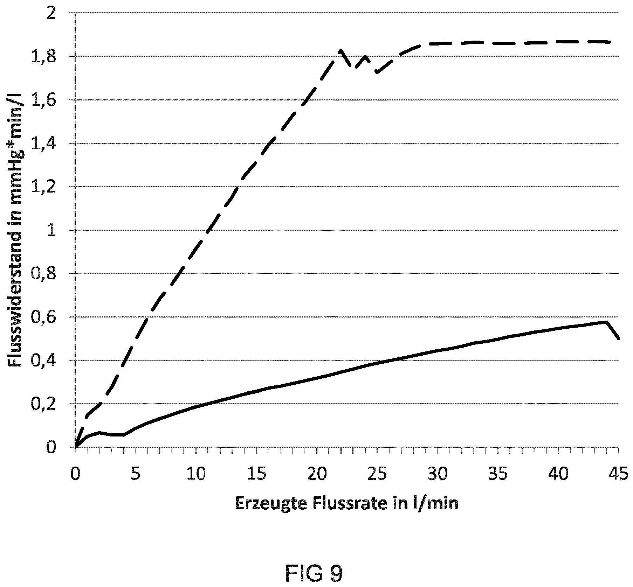

Die Figur 9 zeigt schließlich eine graphische Darstellung des Durchflusswiderstands, also des Quotienten aus Staudruck und Durchfluss, gegenüber der erzeugten Flussrate. Wiederum sind die Messwerte, die den aus dem Stand der Technik bekannten männlichen Luer-Lock-Anschluss betreffen, mit einer gestrichelten Kurve dargestellt, während die Messwerte, die das Ausführungsbeispiel des Verbindungselements gemäß der Erfindung betreffen, mit einer durchgezogenen Kurve wiedergegeben sind. Beim neuartigen Verbindungselement steigt der Durchflusswiderstand linear sehr moderat bis zu einer erzeugten Flussrate von etwa 44 Litern pro Minute an. Demgegenüber kommt es bei dem aus dem Stand der Technik bekannten System bis zu einer Flussrate von etwa 22 Litern pro Minute zu einem steilen Anstieg, der dann in einen linearen Plateau-artigen Verlauf übergeht. Insgesamt ist der Durchflusswiderstand des neuartigen Systems signifikant geringer als der Durchflusswiderstand des aus dem Stand der Technik bekannten Systems. Damit eignet sich das vorliegend beschriebene Verbindungselement hervorragend zur fluiddichten Verbindung eines Schlauches oder eines anderen Hohlkörpers mit einem Schlauch oder einem anderen Hohlkörper bei gleichzeitiger Ermöglichung eines sehr hohen Durchflusses unter Ausbildung eines lediglich geringen Staudrucks. Ferner ist das vorliegend beschriebene Verbindungselement einfach zu bedienen und lässt sich als Spritzgussteil herstellen.

## Patentansprüche

1. Verwendung eines Verbindungselementes, das zur Verbindung zweier von einem Fluid durchströmbarer Hohlkörper geeignet ist und ein erstes Verbindungsteil (2) aufweist, das in fluiddichte Strömungsverbindung mit einem ersten von einem Fluid durchströmbaren Hohlkörper gebracht werden kann, zur Aufnahme eines weiblichen Luer-Lock-Anschlusses als zweitem Verbindungsteil (13) in einem Innenbereich des ersten Verbindungsteils (2),
wobei das Verbindungselement ferner folgendes aufweist:
ein Dichtelement (3), das zumindest teilweise in dem Innenbereich des ersten Verbindungsteils (2) angeordnet ist, und
ein Sicherungselement (4), das an einer Außenseite des ersten Verbindungsteils angeordnet ist,
wobei das Sicherungselement (4) von einer entsicherten Stellung in eine gesicherte Stellung überführt werden kann,
wobei das Sicherungselement (4) in der gesicherten Stellung derart auf das Dichtelement (3) einwirkt, dass ein innerer Durchmesser (d) des Dichtelements (3) kleiner ist als ein erster Durchmesser (D) des Dichtelements (3), wenn sich das Sicherungselement (4) in der entsicherten Stellung befindet, so dass eine fluiddichte Verbindung zwischen dem Innenbereich des ersten Verbindungsteils (2) und einem Innenbereich eines zweiten Verbindungsteils (13) resultiert, wenn das zweite Verbindungsteil (13) in dem Innenbereich des ersten Verbindungsteils (2) aufgenommen ist,
wobei in der gesicherten Stellung eine Dichtung über die Außenseite des weiblichen Luer-Lock-Anschlusses realisiert wird, wenn der weibliche Luer-Lock-Anschluss in dem Innenbereich des ersten Verbindungsteils (2) aufgenommen ist.

2. Verwendung nach Anspruch 1, **dadurch gekennzeichnet, dass** das Sicherungselement (4) das erste Verbindungsteil (2) zumindest abschnittsweise überdeckt.

3. Verwendung nach Anspruch 1 oder 2, **dadurch gekennzeichnet, dass** das Sicherungselement (4) einen gesamten Außenumfang des ersten Verbindungsteils (2) zumindest abschnittweise überdeckt.

4. Verwendung nach einem der vorhergehenden Ansprüche, **dadurch gekennzeichnet, dass** das erste Verbindungsteil (2) und das Sicherungselement (4) über ein Gewinde (7) miteinander in Eingriff stehen, wobei eine Bewegung des Sicherungselements (4) entlang des Gewindes (7) eine Überführung des Sicherungselements (4) von der entsicherten Stellung in die gesicherte Stellung bewirkt.

5. Verwendung nach einem der vorhergehenden Ansprüche, **dadurch gekennzeichnet, dass** das Sicherungselement (4) auch von der gesicherten Stellung in die entsicherte Stellung überführt werden kann.

6. Verwendung nach einem der vorhergehenden Ansprüche, **dadurch gekennzeichnet, dass** das Sicherungselement (4) in seiner gesicherten Stellung eine Klemmkraft auf das Dichtelement (3) und ein in dem Innenbereich des ersten Verbindungsteils (2) aufgenommenes zweites Verbindungsteil (13) ausübt.

7. Verwendung nach einem der vorhergehenden Ansprüche, **dadurch gekennzeichnet, dass** das Sicherungselement (4) zumindest abschnittsweise eine konisch ausgebildete Ausgestaltung (12) aufweist.

8. Verwendung nach einem der vorhergehenden Ansprüche, **dadurch gekennzeichnet, dass** das Sicherungselement (4) bei bestimmungsgemäßer Benutzung des Verbindungselements (1) stets mit dem ersten Verbindungsteil (2) verbunden ist und nicht unbeabsichtigt von dem ersten Verbindungsteil (2) entfernt werden kann.

9. Verwendung nach einem der vorhergehenden Ansprüche, **dadurch gekennzeichnet, dass** ein Abschnitt (11) des Dichtelements (3) außerhalb des Innenbereichs des ersten Verbindungsteils (2) angeordnet ist.

10. Verwendung nach Anspruch 9, **dadurch gekennzeichnet, dass** der außerhalb des Innenbereichs des ersten Verbindungsteils (2) angeordnete Abschnitt (11) des Dichtelements (3) vom Sicherungselement (4) in dessen gesicherter Stellung direkt kontaktiert wird.

11. Verwendung nach Anspruch 9 oder 10, **dadurch gekennzeichnet, dass** der außerhalb des Innenbereichs des ersten Verbindungsteils (2) angeordnete Abschnitt (11) des Dichtelements (3) dafür vorgesehen und eingerichtet ist, einen Vorsprung (16) eines in dem Innenbereich des ersten Verbindungsteils (2) aufgenommenen zweiten Verbindungsteils (13) hinterschnittartig zu umgreifen.

12. Verwendung nach einem der vorhergehenden Ansprüche, **dadurch gekennzeichnet, dass** ein kleinster innerer Durchmesser des ersten Verbindungsteils (2) 3 mm bis 15 mm beträgt.

13. Verbindungsanordnung, mit einem Verbindungselement (1) zur Verbindung zweier von einem Fluid durchströmbarer Hohlkörper, mit
einem ersten Verbindungsteil (2), das in fluiddichte Strömungsverbindung mit einem ersten von einem Fluid durchströmbaren Hohlkörper gebracht werden kann,
einem Dichtelement (3), das zumindest teilweise in einem Innenbereich des ersten Verbindungsteils (2) angeordnet ist,
einem in dem Innenbereich des ersten Verbindungsteils (2) aufgenommenen zweiten Verbindungsteils (13), das in fluiddichte Strömungsverbindung mit einem zweiten von einem Fluid durchströmbaren Hohlkörper gebracht werden kann, und
einem Sicherungselement (4), das an einer Außenseite des ersten Verbindungsteils angeordnet ist,
wobei das Sicherungselement (4) von einer entsicherten Stellung in eine gesicherte Stellung überführt werden kann,
wobei das Sicherungselement (4) in der gesicherten Stellung derart auf das Dichtelement (3) einwirkt, dass ein innerer Durchmesser (d) des Dichtelements (3) kleiner ist als ein erster Durchmesser (D) des Dichtelements (3), wenn sich das Sicherungselement (4) in der entsicherten Stellung befindet, so dass eine fluiddichte Verbindung zwischen dem Innenbereich des ersten Verbindungsteils (2) und einem Innenbereich eines zweiten Verbindungsteils (13) resultiert,
**dadurch gekennzeichnet,**
**dass** das zweite Verbindungsteil (13) ein weiblicher Luer-Lock-Anschluss ist.

## Claims

1. Use of a connecting element that is suitable for connecting two hollow bodies through which a fluid can flow and comprises a first connecting part (2) that can be brought into a fluid-tight flow connection with a first hollow body through which a fluid can flow, for receiving a female Luer Lock connector as a second connecting part (13) in an inner region of the first connecting part (2),
wherein the connecting element furthermore comprises:
a sealing element (3) that is at least partly arranged in the inner region of the first connecting part (2), and
a securing element (4) that is arranged on an outer side of the first connecting part,
wherein the securing element (4) can be transferred from a non-secured position into a secured position,
wherein in the secured position the securing element (4) acts on the sealing element (3) in such a way that an inner diameter (d) of the sealing element (3) is smaller than a first diameter (D) of the sealing element (3) when the securing element (4) is in the non-secured position, which results in a fluid-tight connection between the inner region of the first connecting part (2) and an inner region of a second connecting part (13) when the second connecting part (13) is received in the inner region of the first connecting part (2),
wherein in the secured position a seal is realized via the outer side of the female Luer Lock connector, when the female Luer Lock connector is received in the inner region of the first connecting part (2).

2. Use according to claim 1, **characterized in that** the securing element (4) at least sectionally covers the first connecting part (2).

3. Use according to claim 1 or 2, **characterized in that** the securing element (4) at least sectionally covers an entire outer circumference of the first connecting part (2).

4. Use according to any of the preceding claims, **characterized in that** the first connecting part (2) and the securing element (4) are in engagement with each other via a thread (7), wherein a movement of the securing element (4) along the thread (7) effects a transfer of the securing element (4) from the non-secured position into the secured position.

5. Use according to any of the preceding claims, **characterized in that** the securing element (4) can also be transferred from the secured position into the non-secured position.

6. Use according to any of the preceding claims, **characterized in that** in its secured position the securing element (4) exerts a clamping force on the sealing element (3) and on a second connecting part (13) received in the inner region of the first connecting part (2).

7. Use according to any of the preceding claims, **characterized in that** the securing element (4) at least sectionally has a conically formed design (12).

8. Use according to any of the preceding claims, **characterized in that** when the connecting element (1) is properly utilized, the securing element (4) always is connected with the first connecting part (2) and cannot inadvertently be removed from the first connecting part (2).

9. Use according to any of the preceding claims, **characterized in that** a portion (11) of the sealing element (3) is arranged outside the inner region of the first connecting part (2).

10. Use according to claim 9, **characterized in that** the portion (11) of the sealing element (3) arranged outside the inner region of the first connecting part (2) is directly contacted by the securing element (4) in its secured position.

11. Use according to claim 9 or 10, **characterized in that** the portion (11) of the sealing element (3) arranged outside the inner region of the first connecting part (2) is provided and adapted to enclose a protrusion (16) of a second connecting part (13) received in the inner region of the first connecting part (2) in the manner of an undercut.

12. Use according to any of the preceding claims, **characterized in that** a smallest inner diameter of the first connecting part (2) is 3 mm to 15 mm.

13. A connecting assembly, with a connecting element (1) for connecting two hollow bodies through which a fluid can flow, comprising
a first connecting part (2) that can be brought into fluid-tight flow connection with a first hollow body through which a fluid can flow,
a sealing element (3) that is at least partly arranged in an inner region of the first connecting part (2),
a second connecting part (13) received in the inner region of the first connecting part (2), which can be brought into fluid-tight flow connection with a second hollow body through which a fluid can flow, and
a securing element (4) that is arranged on an outer side of the first connecting part,
wherein the securing element (4) can be transferred from a non-secured position into a secured position,
wherein in the secured position the securing element (4) acts on the sealing element (3) in such a way that an inner diameter (d) of the sealing element (3) is smaller than a first diameter (D) of the sealing element (3) when the securing element (4) is in the non-secured position, which results in a fluid-tight connection between the inner region of the first connecting part (2) and an inner region of a second connecting part (13),
**characterized in**
**that** the second connecting part (13) is a female Luer Lock connector.

## Revendications

1. Utilisation d'un élément de liaison qui est adapté pour relier deux corps creux pouvant être traversés par un fluide et qui présente une première pièce de liaison (2) qui peut être mise en liaison d'écoulement étanche aux fluides avec un premier corps creux pouvant être traversé par un fluide, pour recevoir un raccord Luer-Lock femelle comme deuxième pièce de liaison (13) dans une zone intérieure de la première pièce de liaison (2),
l'élément de liaison comprenant en outre :
un élément d'étanchéité (3) qui est disposé au moins partiellement dans la zone intérieure de la première pièce de liaison (2), et
un élément de sécurité (4) qui est disposé sur un côté extérieur de la première pièce de liaison,
dans lequel l'élément de sécurité (4) peut être transféré d'une position déverrouillée à une position verrouillée,
dans lequel l'élément de sécurité (4) agit sur l'élément d'étanchéité (3) dans la position verrouillée de telle sorte qu'un diamètre intérieur (d) de l'élément d'étanchéité (3) est inférieur à un premier diamètre (D) de l'élément d'étanchéité (3) lorsque l'élément de sécurité (4) est dans la position déverrouillée, de sorte qu'il en résulte une liaison étanche aux fluides entre la zone intérieure de la première pièce de liaison (2) et une zone intérieure de la deuxième pièce de liaison (13) lorsque la deuxième pièce de liaison (13) est reçue dans la zone intérieure de la première pièce de liaison (2),
dans lequel, dans la position verrouillée, un joint d'étanchéité est réalisé par l'intermédiaire du côté extérieur du raccord Luer-Lock femelle lorsque le raccord Luer-Lock femelle est reçu dans la zone intérieure de la première pièce de liaison (2).

2. Utilisation selon la revendication 1, **caractérisée en ce que** l'élément de sécurité (4) recouvre au moins partiellement la première pièce de liaison (2).

3. Utilisation selon la revendication 1 ou 2, **caractérisée en ce que** l'élément de sécurité (4) recouvre au moins partiellement toute une circonférence extérieure de la première pièce de liaison (2).

4. Utilisation selon l'une quelconque des revendications précédentes, **caractérisée en ce que** la première pièce de liaison (2) et l'élément de sécurité (4) sont en prise l'un avec l'autre par l'intermédiaire d'un filetage (7), un mouvement de l'élément de sécurité (4) le long du filetage (7) provoquant un transfert de l'élément de sécurité (4) de la position déverrouillée à la position verrouillée.

5. Utilisation selon l'une quelconque des revendications précédentes, **caractérisée en ce que** l'élément de sécurité (4) peut également être transféré de la position verrouillée à la position déverrouillée.

6. Utilisation selon l'une quelconque des revendications précédentes, **caractérisée en ce que** l'élément de sécurité (4), dans sa position verrouillée, exerce une force de serrage sur l'élément d'étanchéité (3) et une deuxième pièce de liaison (13) reçue dans la zone intérieure de la première pièce de liaison (2).

7. Utilisation selon l'une quelconque des revendications précédentes, **caractérisée en ce que** l'élément de sécurité (4) présente au moins partiellement une configuration (12) de forme conique.

8. Utilisation selon l'une quelconque des revendications précédentes, **caractérisée en ce que** l'élément de sécurité (4) est toujours relié à la première pièce de liaison (2) lorsque l'élément de liaison (1) est utilisé conformément à sa destination et ne peut pas être retiré de la première pièce de liaison (2) par inadvertance.

9. Utilisation selon l'une quelconque des revendications précédentes, **caractérisée en ce qu'**une partie (11) de l'élément d'étanchéité (3) est située à l'extérieur de la zone intérieure de la première pièce de liaison (2).

10. Utilisation selon la revendication 9, **caractérisée en ce que** la partie (11) de l'élément d'étanchéité (3) disposée à l'extérieur de la zone intérieure de la première pièce de liaison (2) est directement contactée par l'élément de sécurité (4) dans sa position verrouillée.

11. Utilisation selon la revendication 9 ou 10, **caractérisée en ce que** la partie (11) de l'élément d'étanchéité (3), disposée à l'extérieur de la zone intérieure de la première pièce de liaison (2), est prévue et agencée pour entourer en contre-dépouille une saillie (16) d'une deuxième pièce de liaison (13) reçue dans la zone intérieure de la première pièce de liaison (2).

12. Utilisation selon l'une quelconque des revendications précédentes, **caractérisée en ce qu'**un plus petit diamètre intérieur de la première pièce de liaison (2) est compris entre 3 mm et 15 mm.

13. Ensemble de liaison, avec un élément de liaison (1) pour relier deux corps creux pouvant être traversés par un fluide, comprenant
une première pièce de liaison (2) qui peut être mise en liaison d'écoulement étanche aux fluides avec un premier corps creux pouvant être traversé par un fluide,
un élément d'étanchéité (3), qui est disposé au moins partiellement dans une zone intérieure de la première pièce de liaison (2),
une deuxième pièce de liaison (13) reçue dans la zone intérieure de la première pièce de liaison (2), qui peut être mise en liaison d'écoulement étanche aux fluides avec un deuxième corps creux pouvant être traversé par un fluide, et
un élément de sécurité (4) qui est disposé sur un côté extérieur de la première pièce de liaison,
dans lequel l'élément de sécurité (4) peut être transféré d'une position déverrouillée à une position verrouillée,
dans lequel l'élément de sécurité (4) agit sur l'élément d'étanchéité (3) dans la position verrouillée de telle sorte qu'un diamètre intérieur (d) de l'élément d'étanchéité (3) est inférieur à un premier diamètre (D) de l'élément d'étanchéité (3) lorsque l'élément de sécurité (4) est dans la position déverrouillée, de sorte qu'il en résulte une liaison étanche aux fluides entre la zone intérieure de la première pièce de liaison (2) et une zone intérieure d'une deuxième pièce de liaison (13),
**caractérisé en ce que**
la deuxième pièce de liaison (13) est un raccord Luer-Lock femelle.
